# EUROPEAN PATENT APPLICATION

(11) **EP 4 257 957 A1**
(43) Date of publication of application: **11.10.2023**
(21) Application number: 22167088.8
(22) Date of filing: 07.04.2022
(51) Int. Cl.: G01N 21/64, G01N 21/84, G01N 21/77, G01N 33/18, G01N 35/00

(54) **CHEMOSENSOR, USE THEREOF, AND METHOD OF DETECTING IONS**

(71) Applicant: Robert Bosch GmbH, 70442 Stuttgart (DE)
(72) Inventor: Kornbluth, Mordechai, Brighton, Massachusetts, 02135 (US); Sagar, Kaushal, 787082 Singapore (SG); Pant, Shilpa, 659246 Singapore (SG); Ah Qune, Lloyd, 460037 Singapore (SG)

(57) **Abstract**

Aspects concern a chemosensor device (100) comprising an optoelectrical transducer comprising a light source (104); a sensor layer (106) placed in the optoelectrical transducer, the sensor layer comprising a plurality of compounds, each of the plurality of compounds associated with an ionophore moiety and fluorophore moiety couple; a multiplexer (110) arranged to operatively select at least one of the compounds to be illuminated by the light source and obtain a fluorescent measurement associated with the at least one compound.

## Description

### TECHNICAL FIELD

This disclosure relates to a chemosensor which is particularly suited for detection of multiple ion types in water.

### BACKGROUND

Fluorescence-based chemosensors can be used for water quality monitoring by detecting trace quantities of heavy metallic and mineral ions. Non-limiting examples of different ion types include lead (Pb) ions, arsenic (As) ions, mercury (Hg) ions, calcium (Ca) ions, magnesium (Mg) ions, sodium (Na) ions, etc. A typical chemosensor device includes an ion-specific receptor molecule coupled to a fluorophore by a spacer molecule, and its operation is based on photoinduced electron transfer principles. These chemosensor molecules are typically developed on polyethylene films as known solutions for sensing of ions. Such polyethylene films may be referred to as chemosensor films.

An optical chemosensor typically consists of an immobilized chemical (chemoreceptor) that responds optically to the analyte, a compatible light source and an optical detector.

However, each chemosensor device is merely suited for the detection of a single ion type. If multiple ions are to be detected, multiple chemosensor devices will have to be deployed. Such configuration suffers from increased costs. In addition, multiple chemosensor devices take up additional space and result in duplicates in components.

There exists a need to provide a solution to address at least one of the aforementioned problems.

### SUMMARY

This disclosure was conceptualized for the detection of multiple types of ions, requiring different chemosensor films to be used. A relatively compact solution that uses a single optical system (LED and photodetector) for multiple chemosensor films in a multiplexed manner for the detection of multiple ion-types is provided. The multiplexing arrangement further supports inline calibration of the optical system.

According to the present disclosure, a chemosensor device as claimed in claim 1 is provided.

The dependent claims define some examples associated with the chemosensor device.

### BRIEF DESCRIPTION OF THE DRAWINGS

The disclosure will be better understood with reference to the detailed description when considered in conjunction with the non-limiting examples and the accompanying drawings, in which:
- FIG. 1A shows a prior art solution of using multiple chemosensor devices for use to detect a plurality of ion types in a water stream requiring multiple optical systems;
- FIG. 1B shows a schematic illustration of the present disclosure requiring a single optical system with a multiplexer;
- FIG. 2 to FIG. 5 show various embodiments of chemosensor devices according to the present disclosure; and
- FIG. 6 and FIG. 7 show flow charts related to methods of detecting a plurality of ion types in a water sample.

### DETAILED DESCRIPTION

The following detailed description refers to the accompanying drawings that show, by way of illustration, specific details and embodiments in which the disclosure may be practiced. These embodiments are described in sufficient detail to enable those skilled in the art to practice the disclosure. Other embodiments may be utilized and structural, and logical changes may be made without departing from the scope of the disclosure. The various embodiments are not necessarily mutually exclusive, as some embodiments can be combined with one or more other embodiments to form new embodiments.

Embodiments described in the context of one of the devices, systems or methods are analogously valid for the other devices, systems or methods.

Features that are described in the context of an embodiment may correspondingly be applicable to the same or similar features in the other embodiments. Features that are described in the context of an embodiment may correspondingly be applicable to the other embodiments, even if not explicitly described in these other embodiments. Furthermore, additions and/or combinations and/or alternatives as described for a feature in the context of an embodiment may correspondingly be applicable to the same or similar feature in the other embodiments.

In the context of various embodiments, the articles "a", "an" and "the" as used with regard to a feature or element include a reference to one or more of the features or elements.

As used herein, the term "and/or" includes any and all combinations of one or more of the associated listed items.

As used herein, the term "chemosensor device" broadly refers to any device that facilitates sensing or detection of at least one ion type. The term broadly includes a molecular sensor that interacts with a specific analyte to produce a detectable change. Such detectable change can include emission of light, that is, fluorescence. The molecular sensor can interact with other hardware and/or software components to form a composite device system. Non-limiting examples of ion types include lead (Pb) ions, arsenic (As) ions, mercury (Hg) ions, calcium (Ca) ions, magnesium (Mg) ions, and sodium (Na) ions, etc.

As used herein, the term "multiplexer" broadly refers to a device, a number of devices, and/or an arrangement of devices that perform selection of an output from a plurality of possible outputs in response to one or more inputs. As an example, a multiplexer may select from a plurality of chemosensor films, each chemosensor film associated with the detection of a specific type of ion, in response to an input generated by a user or by a computer. As a non-limiting example, the multiplexer may include a controller module having a processor with software installed or hard-coded thereon, the controller module arranged in signal communication with one or more physical devices to facilitate selection. Such physical devices may include, but not limited to, electrical motors, stepper motors, shutters, spools, sensors, etc. It is contemplated that various components forming the multiplexer to achieve multiplexing functions may not be physically linked or connected. For example, a remote controller may send a control signal to a motor via a wireless network to align a chemosensor film within an optical path of a fluorescence based chemosensor device.

As used herein, the term "optoelectronic transducer" includes a system for fluorescence-based chemo sensing. The optoelectronic transducer may include a light source, such as a light-emitting diode (LED) and a photo detector. The LED may emit light at various wavelengths, such as a wavelength of 400 to 480 nm. The optoelectronic transducer may include an optical filter, optionally with a narrow band pass, a colored glass filter, optionally with a long pass, an optical analyzer, optionally a photo diode, a microprocessor, and a software interface.

As used herein, the term "module" refers to, or forms part of, or include an Application Specific Integrated Circuit (ASIC); an electronic circuit; a combinational logic circuit; a field programmable gate array (FPGA); a processor (shared, dedicated, or group) that executes code; other suitable hardware components that provide the described functionality; or a combination of some or all of the above, such as in a system-on-chip.

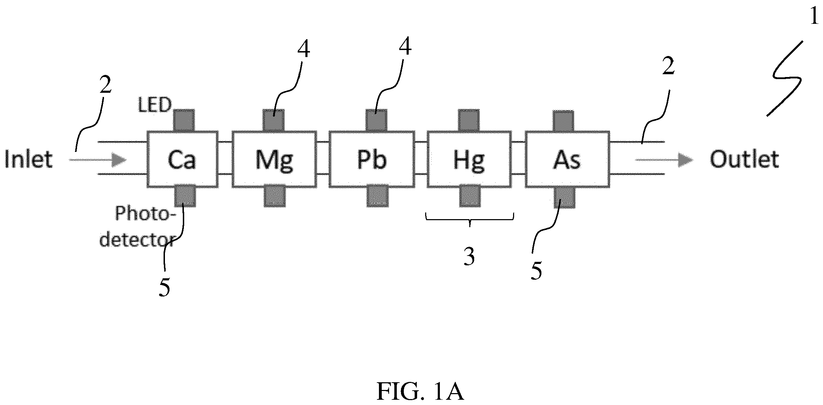

FIG. 1A shows a prior art chemosensor system 1 for detection of multiple types of ions in a water stream 2 with an inlet and outlet. The chemosensor system 1 comprises multiple chemosensor devices 3. Each chemosensor device 3 comprises a LED source 4 and a photodetector 5. As shown, each chemosensor device 3 is configured to detect or sense one particular ion type such as Ca ion, Mg ion, Pb ion, Hg ion, As ion, etc based on photoinduced electron transfer (PET) principles. Each chemosensor comprises an ion-specific receptor molecule (also referred to as an ionophore moiety) which can be coupled to a fluorophore moiety by a spacer molecule. The chemosensor molecules may be developed on ion-specific polyethylene films also referred to as chemosensor films. As may be appreciated, the prior art chemosensor system 1 requires multiple LED source 4 and photodetector 5, and separate chemosensor films for the detection of different ion-types.

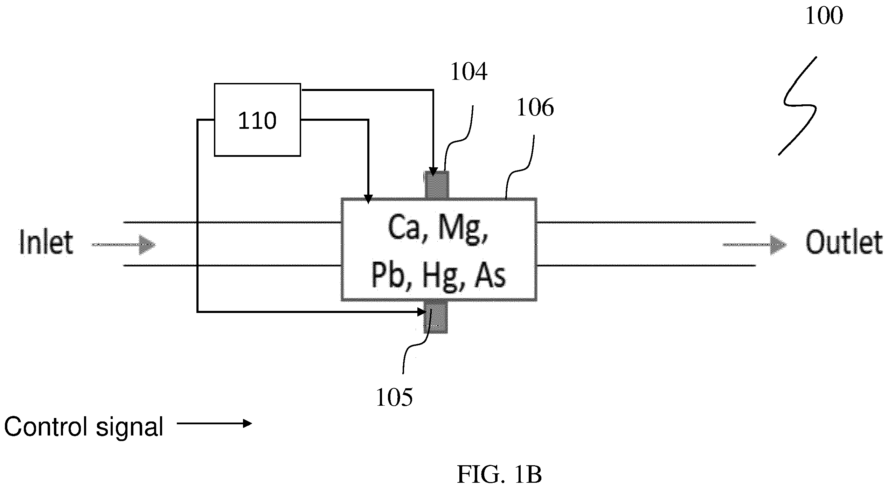

FIG. 1B shows a chemosensor device 100 of the present disclosure. The chemosensor device 100 may be a single device or a system comprising a multiplexer aimed to measure various ion-types of interest. The chemosensor device 100 comprises an optoelectrical transducer comprising a light source 104 and a photodetector 105; a sensor layer 106 placed between the light source 104 and photo detector 105, the sensor layer 106 comprising a plurality of compounds, each of the plurality of compounds associated with an ionophore moiety and fluorophore moiety couple; a multiplexer 110 arranged to operatively select at least one of the compounds to be illuminated by the light source 104 and/or obtain a fluorescent measurement associated with the at least one compound. The selection of each compound for illumination by the multiplexer 110 may be via one or more control signals sent to one or more mechanical devices which may in turn manipulate the light source 104, photodetector 105, the sensor layer 106, and/or other devices not shown.

The fluorophore is a fluorescent moiety which exhibits a particular excitation wavelength and upon excitation, emits at least one fluorescence signal at a higher wavelength. The receptor moiety, or ionophore moiety, is very specific in terms of chelating/binding to a guest molecule or an analyte, such as lead (Pb) ions. An empty receptor site would result in quenching of the fluorescence signal due to photo-induced electron transfer (PET) of an electron from electron rich receptor moiety to the fluorescent moiety. In the event of a lead ion chelating with the ionophore moiety, the PET effect is nullified, as no electron can undergo the PET, resulting in enhanced fluorescence signal. The signal strength, as a fluorescent measurement, is in proportion to the ion concentration and thus can be used for quantitative analysis of the ion when the fluorescent measurement is acquired.

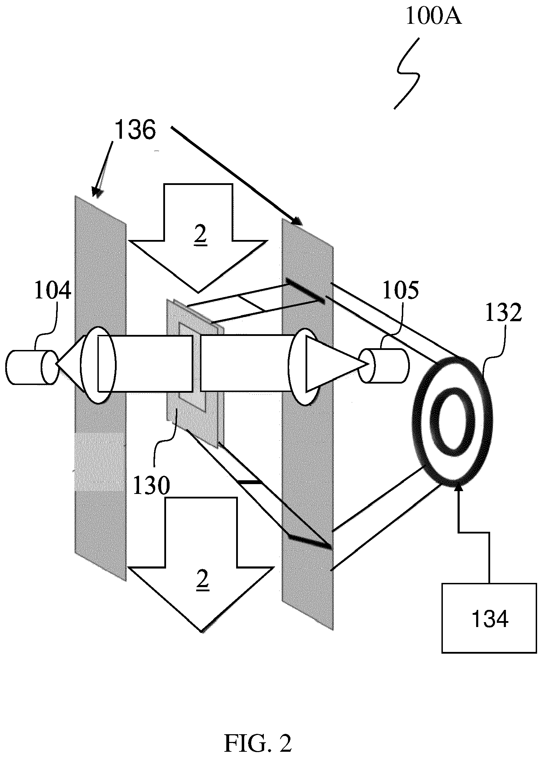

FIG. 2 shows an embodiment of the chemosensor device 100A, wherein the plurality of compounds comprise a plurality of chemosensor films 130. Each chemosensor film 130 may be arranged adjacent another chemosensor film 130. The plurality of chemosensor films 130 may be arranged in a spool or reel 132, and the multiplexer 110 is configured to rotate the spool 132 in a manner so as to position a chemosensor film in the optical path of the light source 104 for irradiation or illumination. Fluorescence measurement may then be acquired by the photodetector 105 of the optical transducer. The chemosensor films 130 may be housed within a casing or opaque housing 136 to prevent or minimize accidental exposure of the films 130 to the light source 104. Optical paths between the light source 104, desired chemosensor film 130 and photo detector 105 may be formed by having at least one aperture on the housing 136 and allowing light to pass through the aperture at relevant intervals.

In some embodiments, the spool device is coupled with a motor 134. The motor 134 may be an electrical motor having controller circuit, operable to receive control signals from the multiplexer 110 to the rotate the spool in a manner so as to position the chemosensor film for illumination.

In some embodiments, the chemosensor device comprises a position sensor to detect the position of the chemosensor film. The position data may be sent back to the multiplexer 110 as a feedback signal or data to determine if the correct chemosensor film is selected in the optical path of the light source 104 and/or if the fluorescence measurement is correctly acquired. The position sensor comprises at least one of a magnetic sensor, RFID tag, microelectronics-based sensor, or some other sensor.

In the embodiment shown in FIG. 2, the number of films that can be multiplexed using the multiplexer is very large. The device 100A can also be used to hold one or more control chemosensor films for calibration purposes. The control chemosensor films may include a dark film and/or a transparent film for purpose of calibration of the optical system which includes at least the light source and photo detector.

In some embodiments, the chemosensor device shown may also include a fluorescent film that is designed to fluoresce under all ion concentrations. This fluorescent film may be the same fluorophore immobilized to the same polymer as the chemosensor films, but lack the receptor that causes photoinduced electron transfer, and therefore will fluoresce regardless of ion content. The fluorophore may be anthracene, benzene, carbazole, diphenylfurane, naphthalene, 1,8-naphthalimide, N,N,N',N'-tetramethyl-benzidine, porphyrin, or pyrene, or any combination thereof.

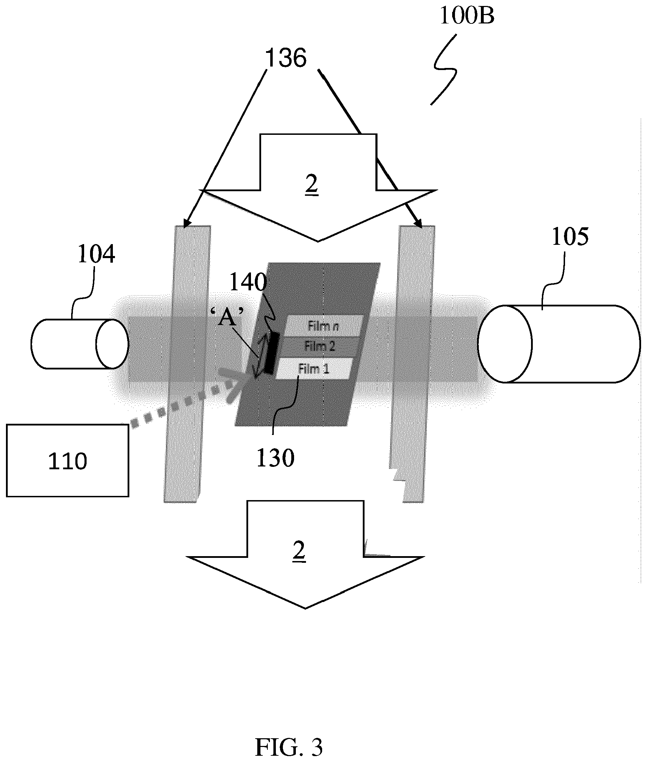

FIG. 3 shows another embodiment of the chemosensor device 100B, having a plurality of chemosensor films 130. Each chemosensor film 130 may be arranged adjacent another chemosensor film 130, and the multiplexer 110 may comprise a shutter mechanism 140 to selectively expose one or more of the plurality of chemosensor films to the light source 104. The shutter mechanism 140 is operable to selectively block the radiation emitted from the light source 104 from reaching a particular chemosensor film 130 that is not selected. The shutter mechanism 140 may be positioned on a side facing the light source 104, rather than on the side of a photodetector, in order to prevent photo-bleaching of the chemosensor films that are not in use. The arrows marked 'A' describe the shutter motion as a sliding movement along the plurality of chemosensor films 130. In operation, an input may be received to expose a chemosensor film 130 associated with the detection of a specific ion (e.g. lead). The shutter mechanism 140 may be movable and moves along the to block other chemosensor films 130 not associated with the lead ion.

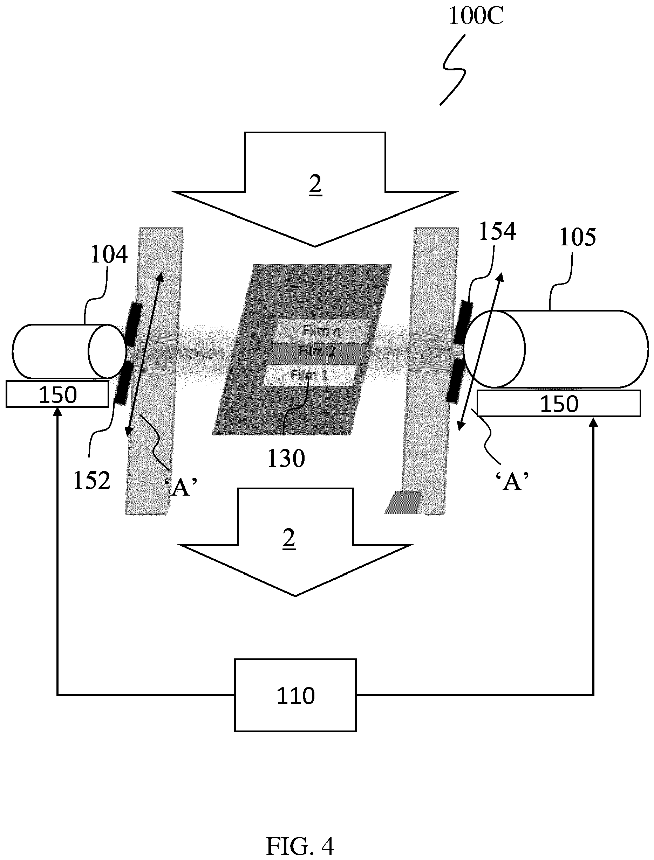

FIG. 4 shows another embodiment of the chemosensor device 100C. The plurality of compounds are developed as a plurality of chemosensor films 130 arranged adjacent each other, the multiplexer 110 comprises a movable mechanism 150 to move the light source 104 and an aperture mechanism 152 mounted on the moveable mechanism 150 in a manner so as to select a chemosensor film 130 for irradiation or illumination by the light source 104. The photodetector 105, with another aperture mechanism 154 may also move in tandem with the light source 104 to acquire the fluorescence measurement. The movement of the light source and photodetector (together with their own apertures) relative to the entire film structure, to ensure that the light reaches only the desired film. In FIG. 4, the arrows 'A' correspond to the light source and photodetector motion. As another embodiment, the chemosensor films 130 and sensor housing can move while the light source 104 and photodetector 105 stay rigid, stationary or in a fixed position.

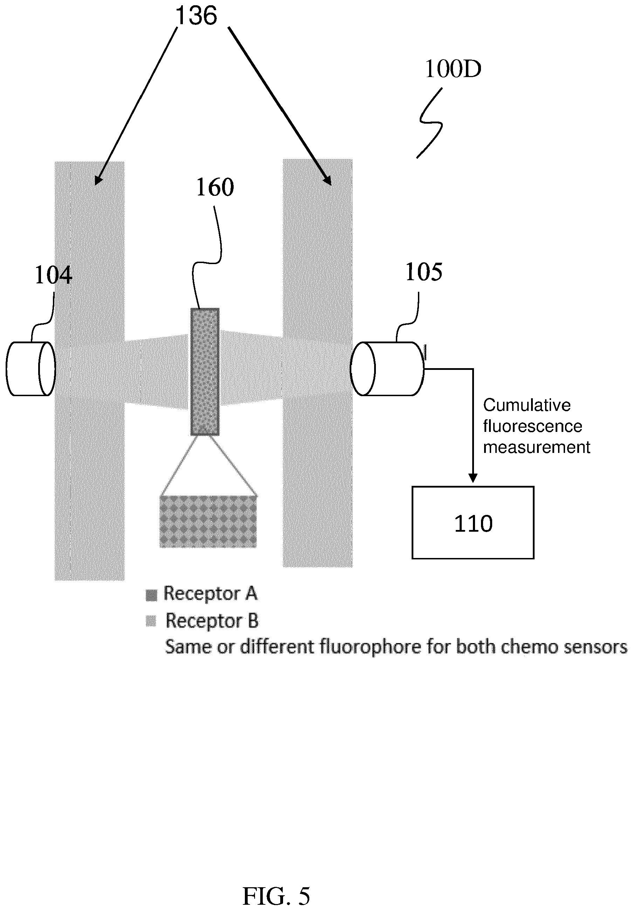

FIG. 5 shows another embodiment of the chemosensor device 100D. Instead of multiple chemosensor films 130 arranged side-by-side one another, the plurality of compounds are integrated into a single chemosensor film 160. The multiplexer 110 may be a processor module configured to obtain a measurement of the cumulative fluorescence associated with at least two different ions, and perform calculations and/or diagnostics based on the cumulative fluorescence measurement, which may be a function of total concentration of the at least two different ions. In other words, two or more chemo sensing molecules may be integrated in a single film. This may be similar to molecular diagnostics multiplexing concept. Depending on the target analyte present in a fluid stream (such as a water stream) where multiple ions are to be detected, same or different fluorophore based chemical structures can be combined in at least one appropriate stoichiometry in a single sensing layer. As an example, to obtain an indication of "water hardness", Calcium (Ca) ion and Magnesium (Mg) ion sensing molecules with same fluorophore can be combined or mixed into a single chemosensor film based on a mixing ratio which may be pre-determined or dynamically determined in use. By adjusting the mixing ratio, the cumulative fluorescence signal collected by the photo detector 105 can be correlated to the total concentration of detected analytes in the fluid stream (e.g. water stream), can be measured and acquired. In this case, as both Ca ion and Mg ion are selectively bound to the respective chemosensor receptor moiety, emitting fluorescence in the same wavelength, cumulative fluorescence is processed using appropriate transfer function(s) to predict water hardness level and not individual ion concentration. Similarly, mixture of different receptor moieties specific to different measurands like Ca ion and Mg ion along with different fluorophores in appropriate stoichiometry can be mixed and different wavelength fluorophores emitting different wavelengths of light can be measured with the photo detector, photo diode module, a spectrometer which covers the entire range for emission peaks for both the fluorophores. Non-limiting examples of transfer functions and derivations of the same, expressed in mathematical equations, are disclosed in the US patent publication US2022/0018775A1, with reference to equations (1) to (14), particularly equations (12) and (13).

The optoelectrical transducer in various described embodiments may comprise a light-emitting diode (LED) as a light source 104, from which an optical pathway passes an optical filter, the sensor layer, and subsequently a colored glass filter and a photo diode functioning as the photo detector 105.

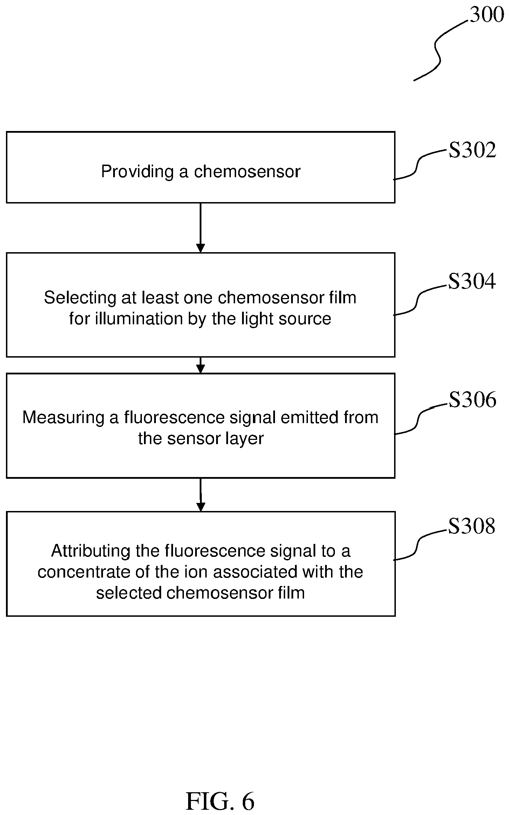

FIG. 6 shows a flow chart of a method 300 for detecting a plurality of ion types in a water sample using the chemosensor device 100, the method 300 comprising the steps of: providing the chemosensor (step S302); selecting at least one chemosensor film for illumination by the light source (step S304); measuring a fluorescence signal emitted from the sensor layer (step S306); and attributing the fluorescence signal to a concentrate of the ion associated with the selected chemosensor film (step S308). The method 300 is suitable for the embodiments shown in FIG. 2 to FIG. 4 involving multiple chemosensor films. The step of selecting at least one chemosensor film for illumination may include sub-steps of moving at least one of the light source, the photo detector, the sensor layer, and one or more light barriers/filters.

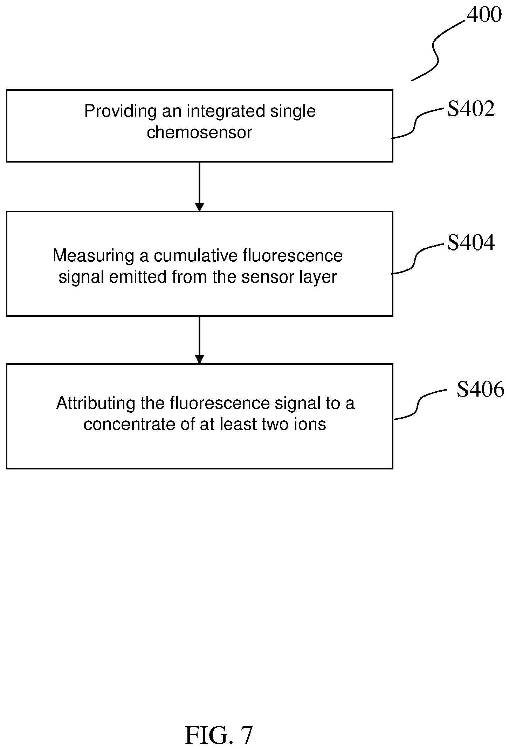

FIG. 7 shows a flow chart of another method 400 for detecting a plurality of ion types in a water sample using the chemosensor device 100. The method 400 comprises the steps of providing an integrated single chemosensor (step S402); measuring a cumulative fluorescence signal emitted from the sensor layer (step S404); and attributing the fluorescence signal to a concentrate of at least two ions (step S406). The method 400 is suitable for the embodiments shown in FIG. 5 involving a single chemosensor film integrating multiple compounds. In some embodiments, the single chemosensor film capable of detecting a plurality of ions may form part of a chemosensor system comprising multiple chemosensor films arranged in a spool.

It is contemplated that the features of various embodiments may be combined. For example, one of the chemosensor film of the plurality of chemosensor films 130 may be an integrated single chemosensor 160. In some embodiments, the spool 132, shutter mechanism 140, moveable mechanism 150 and apertures 152, 154 may be combined in various permutations to form further embodiments not shown.

In some embodiments, a low cost chemosensor can also be realized by using relatively low-cost LED light sources and photodetectors.

While the disclosure has been particularly shown and described with reference to specific embodiments, it may be understood by those skilled in the art that various changes in form and detail may be made therein without departing from the spirit and scope of the disclosure as defined by the appended claims. The scope of the disclosure is thus indicated by the appended claims and all changes which come within the meaning and range of equivalency of the claims are therefore intended to be embraced.

## Claims

1. A chemosensor device (100) comprising
an optoelectrical transducer comprising a light source (104) and a photo detector (105);
a sensor layer (106) placed between the light source and the photo detector, the sensor layer comprising a plurality of compounds, each of the plurality of compounds associated with an ionophore moiety and fluorophore moiety couple;
a multiplexer (110) arranged to operatively select at least one of the compounds to be in an optical path of the light source and obtain a fluorescent measurement associated with the at least one compound.

2. The chemosensor device of claim 1, wherein the plurality of compounds comprise a plurality of chemosensor films (130), each chemosensor film arranged adjacent another chemosensor film arranged in a spool (132), and the multiplexer is configured to rotate the spool in a manner so as to position a chemosensor film in an optical path for irradiation or illumination by the optical transducer.

3. The chemosensor device of claim 2, wherein the spool device is coupled with a motor (134).

4. The chemosensor device of claim 2 or 3, further comprises a position sensor to detect the position of the chemosensor film.

5. The chemosensor device of claim 1, wherein the plurality of compounds is a plurality of chemosensor films, each chemosensor film arranged adjacent another chemosensor film, and wherein the multiplexer comprises a movable shutter (140) to selectively expose one or more of the plurality of chemosensor films to the light source.

6. The chemosensor device of claim 1, wherein the plurality of compounds is a plurality of chemosensor films, and wherein the multiplexer comprises a movable mechanism (150) to move the light source and an aperture mechanism mounted on the moveable mechanism in a manner so as to select a chemosensor film for irradiation or illumination by the optical transducer.

7. The chemosensor device of claim 1, wherein the plurality of compounds are integrated into a single chemosensor film (160), and the multiplexer is configured to obtain a measurement of the cumulative fluorescence associated with at least two different ions.

8. The chemosensor device of any one of the preceding claims, wherein the optoelectrical transducer comprises a light-emitting diode (LED), from which an optical pathway passes an optical filter, the sensor layer, and subsequently a colored glass filter and a photo diode.

9. A method of detecting a plurality of ion types in a fluid sample, the method comprising the steps of:
(i) providing a chemosensor of claim 1;
(ii) selecting at least one chemosensor film for illumination by the light source;
(iii) measuring a fluorescence signal emitted from the sensor layer; and
(iv) attributing the fluorescence signal to a concentrate of the ion associated with the selected chemosensor film.

10. A method of detecting a plurality of ion types in a fluid sample, the method comprising the steps of:
(i) providing a chemosensor of claim 7;
(ii) measuring a cumulative fluorescence signal emitted from the sensor layer; and
(iii) attributing the fluorescence signal to a concentrate of at least two ions.

11. Use of a chemosensor of claims 1 to 7 for detection of at least two different ions in water.

## Amended claims

### Amended claims in accordance with Rule 137(2) EPC.

1. A chemosensor device (100) comprising
an optoelectrical transducer comprising a light source (104) and a photo detector (105);
a sensor layer (106) placed between the light source and the photo detector, the sensor layer comprising a plurality of compounds, each of the plurality of compounds comprises an ionophore moiety and fluorophore moiety couple, the ionophore moiety having a receptor site specific to an analyte, and the ionophore moiety and fluorophore moiety couple configured that an empty receptor site results in quenching of the fluorescence signal due to a photo-induced electron transfer;
a multiplexer (110) arranged to operatively select at least one of the compounds to be in an optical path of the light source and obtain a fluorescent measurement associated with the at least one compound.

2. The chemosensor device of claim 1, wherein the plurality of compounds comprise a plurality of chemosensor films (130), each chemosensor film arranged adjacent another chemosensor film arranged in a spool (132), and the multiplexer is configured to rotate the spool in a manner so as to position a chemosensor film in an optical path for irradiation or illumination by the optical transducer.

3. The chemosensor device of claim 2, wherein the spool device is coupled with a motor (134).

4. The chemosensor device of claim 2 or 3, further comprises a position sensor to detect the position of the chemosensor film.

5. The chemosensor device of claim 1, wherein the plurality of compounds is a plurality of chemosensor films, each chemosensor film arranged adjacent another chemosensor film, and wherein the multiplexer comprises a movable shutter (140) to selectively expose one or more of the plurality of chemosensor films to the light source.

6. The chemosensor device of claim 1, wherein the plurality of compounds is a plurality of chemosensor films, and wherein the multiplexer comprises a movable mechanism (150) to move the light source and an aperture mechanism mounted on the moveable mechanism in a manner so as to select a chemosensor film for irradiation or illumination by the optical transducer.

7. The chemosensor device of claim 1, wherein the plurality of compounds are integrated into a single chemosensor film (160), and the multiplexer is configured to obtain a measurement of the cumulative fluorescence associated with at least two different ions.

8. The chemosensor device of any one of the preceding claims, wherein the optoelectrical transducer comprises a light-emitting diode (LED), from which an optical pathway passes an optical filter, the sensor layer, and subsequently a colored glass filter and a photo diode.

9. A method of detecting a plurality of ion types in a fluid sample, the method comprising the steps of:
(i) providing a chemosensor of claim 1;
(ii) selecting at least one chemosensor film for illumination by the light source;
(iii) measuring a fluorescence signal emitted from the sensor layer; and
(iv) attributing the fluorescence signal to a concentrate of the ion associated with the selected chemosensor film.

10. A method of detecting a plurality of ion types in a fluid sample, the method comprising the steps of:
(i) providing a chemosensor of claim 7;
(ii) measuring a cumulative fluorescence signal emitted from the sensor layer; and
(iii) attributing the fluorescence signal to a concentrate of at least two ions.

11. Use of a chemosensor of claims 1 to 7 for detection of at least two different ions in water.
